# EUROPEAN PATENT SPECIFICATION

(11) **EP 0 495 390 B1**
(45) Date of publication and mention of the grant of the patent: **17.07.1996**
(21) Application number: 92100259.8
(22) Date of filing: 09.01.1992
(51) Int. Cl.: A23J 3/34

(54) **A process for the production of hydrolyzed proteins**
Verfahren zur Herstellung von eines pflanzliches Eiweisshydrolysat
Procédé de préparation d'hydrolysate de protéines

(30) Priority: 14.01.1991 US 641038
(43) Date of publication of application: 22.07.1992
(73) Proprietor: CPC INTERNATIONAL INC., Englewood Cliffs New Jersey 07632-9976 (US)
(72) Inventor: Hamm, Donald J., New Jersey 07974 (US)
(74) Representative: Lederer, Franz, Dr.

(56) References cited:
- EP-A- 0 361 595
- WO-A-88/07822
- PATENT ABSTRACTS OF JAPAN vol. 12, no. 301 (C-521)16 August 1988 & JP-A-63 074 465 ( NISHIMURA NOBUAKI ) 4 April 1988

## Description

### BACKGROUND OF THE INVENTION

### FIELD OF THE INVENTION

This invention relates to a process for the production of hydrolyzed proteins containing no detectable levels of monochlorodihydroxypropanol. The resultant hydrolyzed proteins are clean and bland in flavor and are capable of exhibiting substantial flavor enhancement characteristics.

### DESCRIPTION OF THE PRIOR ART

The preparation of conventional hydrolyzed proteins (HPs), most commonly hydrolyzed vegetable proteins (HVPs), is generally carried out by acid hydrolysis with hydrochloric acid under refluxing conditions, specifically using 6M hydrochloric acid at 109°C and atmospheric pressure. It has been demonstrated that hydrolyzing proteins under these conditions results in the chlorination of glycerol, which is derived from the residual fatty substances present in the crude protein, which produces monochlorodihydroxypropanols (MCDPs) and dichloropropanols (DCPs). As MCDPs and DCPs exhibit questionable properties and characteristics, their presence is not desired in food products. DCPs are readily removed during the evaporation or concentration steps found in standard processes. Unfortunately, MCDPs are not removed but are concentrated in the finished product, and therefore, additional processing steps must be taken to remove the MCDPs from the finished product resulting from conventional procedures.

In a conventional acid hydrolysis process for preparing HPs, the formation of MCDPs and DCPs can be avoided by using sulfuric or phosphoric acid in place of hydrochloric acid. However, the HPs produced by hydrolyzing with sulfuric or phosphoric acid are of an inferior quality, since they generally exhibit a bitter flavor.

The specific problem being addressed is that MCDP is produced during conventional acid hydrolysis from the chlorination of the glycerol derived from the residual fatty substances which are present in crude proteins. As an example, vital wheat gluten, which is approximately 75% protein, contains about 5.0 to 9.5% fat and other lipid materials, and is an abundant source of glycerol in the form of a complex mixture of mono-, di- and tri-glycerides, phospholipids and glycolipids. Numerous factors are believed to effect the formation of MCDP, including the presence of high concentrations of chloride ions, high amounts of excess acid, high refluxing temperatures and long reaction times. Additionally, it is thought that bound glycerol is more active in forming MCDPs than unbound glycerol.

Much is also known about the use of enzymes to hydrolyze proteins, particularly vegetable proteins, for food use, but not for the purpose of flavor enhancement. What is taught in the existing art is generally directed toward producing functionally improved proteins, such as the elimination of bitter peptide formation during enzyme hydrolysis as shown in U.S. Patent No. 4,636,388. Specifically, the patent discloses a low ash protein product which is particularly adapted for enzymatic hydrolysis. A dispersion of protein is gelled and then washed in particulate form in a liquid in order to allow a portion of the non-proteinaceous material to defuse from the gel into the liquid, and then the liquid is separated from the gel. The pretreated product is then hydrolyzed enzymatically, preferably with fungal protease and pancreatin.

U.S. Patent No. 4,757,007, discloses and claims a process for the preparation of hydrolyzed products of soy protein by partially hydrolyzing soy protein with a protease, and then separating the resulting hydrolyzed products by using a 5% aqueous solution of trichloroacetic acid. The portion of hydrolyzed protein with low solubility possesses excellent emulsifying properties, while the one with high solubility possesses excellent foaming properties.

In U.S. Patent No. 3,830,942, a soluble protein product is produced which is particularly useful in highly acidic foods, and an insoluble protein product is prepared which is used in preparing protein enriched bakery goods. The patent discloses the method for producing the two products by forming an aqueous solution of defatted oleaginous seed materials, adjusting the pH of the slurry to the isoelectric point of the seed materials, heating the slurry to elevated temperatures, adding an enzyme to the slurry, agitating the mixture during hydrolysis of the material and thereafter, separating the hydrolyzed and unhydrolyzed portions of the protein product.

Although enzyme hydrolysis and acid hydrolysis are generally separate procedures, one reference has been found which discloses the combination of acid and enzyme hydrolysis to obtain a protein hydrolyzate. In USSR Patent Application No. 442800, a method of obtaining a preparation for parenteral protein feeding is taught. A method is disclosed wherein the raw protein material undergoes enzymatic cleavage, followed by acid hydrolysis with 5.0% sulfuric acid (4.0N), in a carbon dioxide atmosphere. Thereafter, the hydrolyzate is passed through an anion exchange column, treated with aluminum hydroxide and passed through a column containing cation exchange resin. The acid hydrolysis takes place at about 100°C for about seven (7) hours.

EPA 361 595 discloses a process for improving HCl-hydrolyzed protein by subjecting an aqueous solution thereof to hydrolysis (of monochloropropanediols and dichloropropanols) at a pH between 5.5 and 8.0 at a temperature between 20 and 180°C for a period between 10 days to 15 minutes.

The protease decomposition of gluten meal followed by an acid decomposition and neutralization of the product is disclosed in JPA 63 074 465.

Many attempts have been made to produce hydrolyzed protein products which can be used for various purposes. But to date, no process has been taught which produces a hydrolyzed protein with reduced or non-existent levels of MCDP or DCP due to prevention of the production of MCDP and DCP through controlling the parameters of the acid hydrolysis, and which hydrolyzed protein product is additionally capable of exhibiting substantial flavor enhancement characteristics.

### SUMMARY OF THE INVENTION

The present invention relates to the production of hydrolyzed proteins which contain no detectable levels of MCDP. This result is achieved by a unique process which combines two methods of hydrolysis of the protein; mild acid hydrolysis to deamidate and partially hydrolyze the protein, followed by enzymatic hydrolysis to reduce the protein to amino acids and short chained peptides. The hydrolyzates which result from this process may contain substantial amounts of monosodium glutamate, up to 36% w/w of the starting protein, and are therefore capable of exhibiting substantial flavor enhancement character, and should be clean and bland in flavor.

The process for producing hydrolyzed proteins, particularly hydrolyzed vegetable proteins, with no detectable level of MCDP begins with the selective deamidation of a food grade protein by adding it to a hot, dilute aqueous solution of at least one mineral acid. The pH of the resulting deamidated protein is then adjusted to the appropriate level for the particular enzyme to be used in the subsequent step. Thereafter, at least one protease is added to substantially hydrolyze the deamidated protein to small peptides and free amino acids.

When food proteins contain residual lipids and when hydrochloric acid is used, the mildness of the conditions also directly effect the level of MCDP in the final product. The conditions at which the acid hydrolysis or deamidation take place are significantly milder than those used in conventional processes. Specifically, the mild acid hydrolysis is carried out at significantly lower acid concentrations, at lower temperatures and for shorter periods of time than in conventional hydrolysis processes. When sulfuric or phosphoric acid are used in place of hydrochloric acid slightly higher concentrations of acid may be necessary in order to achieve an equivalent degree of deamidation. However, since no chloride is present in the reaction mixture, no chlorinated glyceride species form when lipids remain in the substrate. By controlling the conditions, deamidation preferentially occurs; ie, the amide linkages are hydrolyzed, but the peptide bond hydrolysis is controlled or minimized. It is believed that these conditions, are responsible for the lack of formation of MCDPs in the finished product.

### DETAILED INVENTION

The present process comprises a number of steps for hydrolyzing a protein, particularly a vegetable protein, to result in a product which contains no detectable level of MCDP. The term "no detectable level" as used herein means that there is no detectable level as measured by gas chromotagraphy (GC) with sensitivity to levels as low as 1ppm.

Specifically, a food grade protein is subjected to mild acid hydrolysis by adding it to a hot, dilute solution of a food grade mineral acid. The protein can be any one of the available vegetable proteins, such as, but not limited to, oil seed proteins (soy, peanut, sunflower, cottonseed), grain proteins, leaf proteins, as well as meat, plasma or milk proteins, or any combination thereof. The preferred protein for producing savory flavors with substantial flavor enhancing properties is wheat gluten, a grain protein, due to its high glutamic acid content, present mostly in the form of glutamine. The mild acid hydrolysis is designed to maximize deamidation of the protein and, in the case of hydrochloric acid, minimize the formation of MCDP, through optimization of the level of excess hydrogen ion concentration. Specifically, after titrating the food protein, the hydrogen ion concentration is from about 0.001 molar(M) or pH 3, to about 2.0M, preferably 1.0M and the total chloride concentration should not exceed 2.0M.

The amount of acid required is, in practice, dependent upon the protein level and protein type. The desired protein level varies from about 1 to about 30% w/w, with the preferred protein concentration being from about 5 to about 20%, dependant upon the particular protein type. Examples of food grade acids which can be used include hydrochloric acid, sulphuric acid, phosphoric acid, and any combinations thereof. The deamidation step is carried out at a temperature of from about 50 to about 100°C, preferably about 95°C for a period of no more than two (2) hours, preferably approximately one (1) hour. At this point, the deamidation process is stopped by reducing the reaction temperature to below 50°C. The product can be held in acid form until it is time to proceed to the enzymatic hydrolysis.

The degree of protein hydrolysis after mild acid deamidation is from about 5 to about 30% depending upon the protein and the reaction conditions used. A benefit of the partial hydrolysis is that it may solubilize a substantial portion of the protein, which can be separated out at this point for subsequent enzymatic hydrolysis. Alternately, the entire reaction mixture can be subjected to enzymatic hydrolysis after adjustment of the pH. This can be accomplished by the addition of any known food grade base, with the preferred base being sodium hydroxide.

The resulting partially hydrolyzed protein is then further hydrolyzed by adding to it at least one protease. The protease should contain at least one endoprotease, which can be acidic, neutral or alkaline in form. The protease is chosen dependent upon a number of parameters for the particular enzyme/substrate combination, such as a) the proper pH for the optimum proteolytic activity; b) the peptide bond specificity, which is best suited to meet the end product requirements; and c) the need for debittering the substrate. The preferred enzyme for the preferred protein wheat gluten, is a neutral endoprotease, specifically Prozyme 6 (Amano International Enzyme, Troy, Virgina). Because deamidation converts the majority of the hydrophobic glutamine residues to polar charged glutamic acid residues, a protease which is specific for hydrolyzing next to glutamic acid residues may be useful. For example, the endoprotease from Staphylococcus V8 which specifically hydrolyzes peptide bonds on the carboxyl side of glutamic acid residues is particularly useful.

The enzyme hydrolysis of the deamidate occurs at a temperature of from about 25 to about 75°C and, with a neutral enzyme present in the amount of from about 0.1% to about 2.0% by weight of the substrate at a pH of from about 5.5 to about 8.5. Again, these conditions will vary depending on the protein-protease combination as the pH is dependent upon the type of enzyme used. For example, if an acidic enzyme is used, the pH will be in the range of from about 1.5 to about 4.0 and if an alkaline enzyme is used, the pH will be in the range of from about 7.0 to about 12.0. The pH range first set out above is based on the use of a neutral protease.

For the preferred case of wheat gluten and Prozyme 6, a neutral protease, the enzyme hydrolysis is carried out at a temperature of from about 40 to about 50°C, preferably 45°C, and at a pH of from about 6.0 to about 7.5, with a preferred level from about 0.5% to 1.0% by weight Prozyme 6. The time during which the enzyme hydrolysis takes place is dependent upon quite a number of factors, specifically, the enzyme concentration, the pH, the temperature of the reaction, the substrate level and the desired degree of hydrolysis. For the preferred embodiment, a time period of about four (4) hours is suggested.

The enzyme hydrolysis is designed to complete the peptide bond hydrolysis, which is necessary to release the flavor active peptides and amino acids. It does not release the glutamic acid or monosodium glutamate from glutamine, nor does it act on the amide bonds of the glutamine which is bound to the peptides, which is the function of the deamidation. As stated above, quite a range of commercially available endoproteases and exoproteases may be used to achieve the desired result. Specific exoproteases, such as Prozyme 6 which also contains leucine amino peptidase, may be used if it is desired to reduce the bitterness from hydrophobic peptides which are present in hydrolyzed vegetable proteins.

Since mild acid deamidation tends to cause some peptide bond hydrolysis, any combination of endoproteases and exoproteases, used either simultaneously or sequentially, may be used to bring about the desired level of protein hydrolysis.

At this point, the enzymatic process may be stopped at the desired stage by the addition of acid to the aqueous solution. This step is not a necessary one, although it is part of the preferred process, and the hydrolyzed soluble protein can be separated from the insoluble mass without it. However, addition of a food grade organic or inorganic acid to bring the aqueous solution to a pH of from about 2.0 to about 4.0 will stop the enzymatic reaction, thereby providing precise control of the end point, and providing microbiological stability to the hydrolyzate may also terminate the enzymatic acitivty by other means such as heat, depending upon the enzyme in use. Addition of a food grade acid at the desired time will also provide for better separation of the hydrolyzed soluble protein from any unhydrolyzed insoluble mass that remains. The enzymatic hydrolysis may be stopped by any means possible once the hydrolyzate has reached the desired degree of solubility and degree of hydrolysis. Specifically, the degree of solubility should, for economic reasons, be at least 60%, with a preferred level of at least 90%. The degree of hydrolysis should be in the range of from about 10 to about 90%, preferably about 50% to about 70%.

The hydrolyzed protein is then separated from any insoluble mass by any suitable, conventional method, such as filtration or centrifugation or combinations thereof. The resultant clarified and neutralized hydrolyzate may then be further processed if desired, to get it into a more usable form. The hydrolyzate can be subjected to decolorization and deodorization processes. This is conventionally carried out by the use of activated carbon. The decolorized, deodorized hydrolyzate may then be concentrated. This can be performed by any number of methods currently known, such as spray drying, vacuum tray drying or evaporation, for example by a falling thin film evaporator.

During the mild acid deamidation, substantially all the glutamine present in the protein backbone is converted to glutamic acid. During the enzymatic hydrolysis this glutamic acid is hydrolyzed to free glutamic acid. Therefore, the amount of MSG present in the final product is determined by the deamidation and enzymatic processes followed as well as the substrate used. For example, if the vegetable protein is wheat gluten and the total process goes to full conversion, the level of MSG in the final product can be as high as about 36% w/w of the starting protein.

### EXAMPLE I

This is an example of sequential hydrolysis of a fat containing protein with dilute hydrochloric acid and a protease.

### Mild Acid Deamidation

Protein deamidation was carried out on a 1 kilogram scale at 10% (w/w) of protein substrate (as received). 850.4 grams of deionized water and 49.6 grams of 35.6% HCl (0.5N) were charged into a 2 liter, three necked round bottom flask and heated with stirring to 95°C. 100 grams of vital wheat gluten [whose fat (lipid) content typically varies from 3 to 10% depending upon the source (75% protein on a dry basis)] were charged as a powder (Gem of the West, Manildia Milling Corp.) into the reactor through a side port, over a period of approximately 3-5 minutes. A condenser was placed in the port and the reaction proceeded for a period of 1 hour at a constant temperature of 95°C. The heat source was removed from the reaction vessel, and the reaction mixture was rapidly cooled below 50°C in an ice bath to stop the reaction. The deamidated protein exhibited total deamidation as measured at approximately 101%.

### Enzymatic hydrolysis

Enzymatic hydrolysis of the product of mild acid deamidation was conducted in a 1 liter vessel maintained at a 45±1°C by a constant temperature water bath. Constant pH was maintained via an autotitrator pH stat. The general procedure followed for conducting the enzyme hydrolysis was to charge the reaction vessel with the deamidate (400 grams containing 40 grams of partially hydrolyzed vital wheat gluten). While the vessel and substrate were being brought up to temperature, the pH electrode was standardized and installed in the reaction vessel to equilibrate under agitation to the final reaction temperature and the autotitrator was charged with 4.0M sodium hydroxide. The titrator was set to the target pH (7.5) and the enzyme solution (8% enzyme w/w in deionized water) was prepared. Once the reaction reached the desired temperature, the reaction mixture was titrated to the desired pH (7.5) with agitation. The reaction was then started by the addition of 5 mL of enzyme (0.4 grams of Prozyme 6, Amano International Enzyme Co., Inc. Troy VA. 22974).

After 4 hours, the substrate was removed from the reactor and cooled to standard refrigerator temperature. After acidification to pH 5.0 with concentrated food grade hydrochloric acid (36.5% HCl), the soluble phase was recovered by centrifuging the hydrolyzate for 15 minutes at 16,000 X G. The MCDP level was then measured by a standard GC procedure with the results indicating less than 1ppm MCDP.

### EXAMPLE 2

This is an example of the impact of adding a second protease specific for hydrolysis at glutamic acid residues.

### Mild Acid Deamidation

Mild acid deamidation was carried out on vital wheat gluten as described in Example 1 above.

### Enzymatic hydrolysis

Enzyme hydrolysis of the product of mild acid deamidation was carried out as described in Example 1 except that 0.5% (w/w) of the endo-glu C protease from Staphylococcus V8 (Sigma Chemical Co., St. Louis, MO) was added in addition to Prozyme 6. As expected from Example 1, MCDP was not detected in the final hydrolyzate.

### EXAMPLE 3

This is an example of sequential hydrolysis of fat containing protein with dilute sulfuric acid and a protease.

### Mild Acid Deamidation

Mild acid deamidation was carried out on vital wheat gluten as described in Example 1 except that 0.5M sulfuric acid was used in place of 0.5M HCl (28.0 grams of concentrated sulfuric acid per 1.0 kilogram batch containing 100 grams vital wheat gluten). The deamidated product exhibited a degree of deamidation of approximately 88.0%.

### Enzymatic hydrolysis

Enzymatic hydrolysis of the product of mild acid deamidation was carried out as described in Example 1. MCDP was not analyzed since background chloride levels from the substrate were insignificant and the hydrolyzing acid used used during mild acid deamidation is not hydrochloric acid.

### EXAMPLE 4

This is an example of sequential hydrolysis of a defatted protein with dilute hydrochloric acid and a protease.

### Mild Acid Deamidation

Mild acid deamidation was carried out on isolated soy protein (PP 620, Protein Technologies International, Checkerboard Square, St. Louis, MO) as described in Example 1. The degree of deamidation of the protein was approximately 85.0%.

### Enzymatic hydrolysis

Enzymatic hydrolysis of the product of mild acid deamidation was carried out as described in Example 1. As expected from Example 1, MCDP was not detected at levels of 1ppm or higher in the final hydrolyzate.

## Claims

1. A process for the production of hydrolyzed proteins containing no detectable level of monochlorodihydroxypropanol, which comprises:
(a) heating a food grade protein in a dilute aqueous acid solution to deamidate the protein;
(b) hydrolyzing the deamidated protein in aqueous solution by adding to it at least one protease; and
(c) neutralizing the hydrolyzed deamidate.

2. The process of Claim 1 which further comprises deodorization and decolorization of the hydrolyzate from step (c).

3. The process of claim 2 which further comprises concentrating the deodorized and decolorized hydrolyzate from step (c).

4. The process of Claim 1 wherein the acid in step (a) is a food grade mineral acid.

5. The process of Claim 4 wherein the food grade mineral acid is chosen from the group consisting of hydrochloric acid, phosphoric acid, sulfuric acid and combinations thereof.

6. The process of Claim 1 wherein the food grade protein in step (a) is chosen from the group consisting of oil seed proteins, grain proteins, plasma proteins, leaf proteins, milk proteins, meat proteins or combinations thereof.

7. The process of Claim 1 wherein the protein added in step 1 is from about 1 to about 30% by weight.

8. The process of Claim 7 wherein the protein concentration is about 5 to about 20% by weight of the reaction mixture.

9. The process of Claim 6 wherein the acid concentration is from 0.001 to 2.0 molar.

10. The process of Claim 9 wherein the acid concentration is 1.0 molar.

11. The process of Claim 1 wherein the reaction in step (a) takes place at a temperature of from about 50 to 100°C.

12. The process of Claim 11 wherein the reaction in step (a) takes place at a temperature of about 95°C.

13. The process of Claim 1 wherein the protease in step (b) is an endoprotease.

14. The process of Claim 13 wherein the endoprotease is an acidic, neutral or alkaline protease.

15. The process of Claim 14 wherein the pH of the deamidated protein in step (a) is adjusted prior to hydrolysis.

16. The process of claim 15 wherein the pH is adjusted to from about 5.5 to about 8.5.

17. The process of Claim 1 wherein the hydrolysis in step (b) is effected by the sequential addition of at least two proteases.

18. The process of Claim 1 wherein the hydrolysis in step (b) is effect by the simultaneous addition of at least two proteases.

19. The process of Claim 18 wherein at least one of the proteases is an exopeptidase.

20. The process of Claim 2 wherein the deodorization and decolorization is carried out by the use of activated carbon.

21. The process of Claim 6 wherein the food grade protein is a defatted protein to eliminate MCDP formation in the presence of hydrochloric acid.

22. The process of Claim 1 wherein the food grade acid is chosen from the group consisting of hydrochloric, sulfuric or phosphoric acid.

## Patentansprüche

1. Verfahren zur Herstellung von hydrolysierten Proteinen, die keinen nachweisbaren Anteil an Monochlordihydroxypropanol enthalten, umfassend
(a) Erhitzen eines Proteins mit Lebensmittelreinheit in einer verdünnten, wässerigen Säurelösung zur Desamidierung des Proteins;
(b) Hydrolysieren des desamidierten Proteins in wässeriger Lösung durch Zugabe mindestens einer Protease dazu; und
(c) Neutralisieren des hydrolysierten Desamidats.

2. Verfahren nach Anspruch 1, das zusätzlich Desodorierung und Entfärbung des Hydrolysats von Schritt (c) umfaßt.

3. Verfahren nach Anspruch 2, das zusätzlich Einengen des desodorierten und entfärbten Hydrolysats von Schritt (c) umfaßt.

4. Verfahren nach Anspruch 1, wobei die Säure in Schritt (a) eine Mineralsäure mit Lebensmittelreinheit ist.

5. Verfahren nach Anspruch 4, wobei die Mineralsäure mit Lebensmittelreinheit ausgewählt ist aus der Gruppe, bestehend aus Salzsäure, Phosphorsäure, Schwefelsäure und Kombinationen davon.

6. Verfahren nach Anspruch 1, wobei das Protein mit Lebensmittelreinheit in Schritt (a) ausgewählt ist aus der Gruppe, bestehend aus Ölsaatproteinen, Getreideproteinen, Plasmaproteinen, Blattproteinen, Milchproteinen, Fleischproteinen oder Kombinationen davon.

7. Verfahren nach Anspruch 1, wobei das Protein, das in Schritt 1 zugegeben wird, etwa 1 bis etwa 30 Gewichtsprozent ausmacht.

8. Verfahren nach Anspruch 7, wobei die Proteinkonzentration etwa 5 bis etwa 20 Gewichtsprozent des Reaktionsgemisches ausmacht.

9. Verfahren nach Anspruch 6, wobei die Säurekonzentration 0,001 bis 2,0 molar ist.

10. Verfahren nach Anspruch 9, wobei die Säurekonzentration 1,0 molar ist.

11. Verfahren nach Anspruch 1, wobei die Reaktion in Schritt (a) bei einer Temperatur von etwa 50 bis 100°C stattfindet.

12. Verfahren nach Anspruch 11, wobei die Reaktion in Schritt (a) bei einer Temperatur von etwa 95°C stattfindet.

13. Verfahren nach Anspruch 1, wobei die Protease in Schritt (b) eine Endoprotease ist.

14. Verfahren nach Anspruch 13, wobei die Endoprotease eine saure, neutrale oder alkalische Protease ist.

15. Verfahren nach Anspruch 14, wobei der pH-Wert des desamidierten Proteins in Schritt (a) vor der Hydrolyse eingestellt wird.

16. Verfahren nach Anspruch 15, wobei der pH-Wert auf etwa 5,5 bis etwa 8,5 eingestellt wird.

17. Verfahren nach Anspruch 1, wobei die Hydrolyse in Schritt (b) durch aufeinanderfolgende Zugabe von mindestens zwei Proteasen bewirkt wird.

18. Verfahren nach Anspruch 1, wobei die Hydrolyse in Schritt (b) durch gleichzeitige Zugabe von mindestens zwei Proteasen bewirkt wird.

19. Verfahren nach Anspruch 18, wobei mindestens eine der Proteasen eine Exopeptidase ist.

20. Verfahren nach Anspruch 2, wobei die Desodorierung und Entfärbung unter Verwendung von Aktivkohle ausgeführt wird.

21. Verfahren nach Anspruch 6, wobei zur Eindämmung der MCDP-Bildung in Gegenwart von Salzsäure das Protein mit Lebensmittelreinheit ein entfettetes Protein darstellt.

22. Verfahren nach Anspruch 1, wobei die Säure mit Lebensmittelreinheit ausgewählt ist aus der Gruppe, bestehend aus Salzsäure, Schwefelsäure oder Phosphorsäure.

## Revendications

1. Procédé de production de protéines hydrolysées ne renfermant aucune quantité détectable de monochlorodihydroxypropanol, qui comprend :
(a) le chauffage d'une protéine de qualité alimentaire dans une solution aqueuse d'acide diluée pour désamider la protéine ;
(b) l'hydrolyse de la protéine désamidée en solution aqueuse par addition à cette solution d'au moins une protéase ; et
(c) la neutralisation du produit de désamidation hydrolysé.

2. Procédé suivant la revendication 1, qui comprend en outre une désodorisation et une décoloration de l'hydrolysat de l'étape (c).

3. Procédé suivant la revendication 2, qui comprend en outre la concentration de l'hydrolysat désodorisé et décoloré de l'étape (c).

4. Procédé suivant la revendication 1, dans lequel l'acide de l'étape (a) est un acide minéral de qualité alimentaire.

5. Procédé suivant la revendication 4, dans lequel l'acide minéral de qualité alimentaire est choisi dans le groupe consistant en acide chlorhydrique, acide phosphorique, acide sulfurique et leurs associations.

6. Procédé suivant la revendication 1, dans lequel la protéine de qualité alimentaire de l'étape (a) est choisie dans le groupe consistant en protéines de graines oléagineuses, protéines de céréales, protéines plasmatiques, protéines de feuilles, protéines de lait, protéines de viande ou leurs associations.

7. Procédé suivant la revendication 1, dans lequel la protéine ajoutée dans l'étape 1 est présente en une quantité d'environ 1 à environ 30 % en poids.

8. Procédé suivant la revendication 7, dans lequel la concentration en protéine est comprise dans l'intervalle d'environ 5 à environ 20 % en poids du mélange réactionnel.

9. Procédé suivant la revendication 6, dans lequel la concentration en acide est comprise dans l'intervalle de 0,001 à 2,0 molaire.

10. Procédé suivant la revendication 9, dans lequel la concentration d'acide est égale à 1,0 molaire.

11. Procédé suivant la revendication 1, dans lequel la réaction dans l'étape (a) s'effectue à une température d'environ 50 à 100°C.

12. Procédé suivant la revendication 11, dans lequel la réaction dans l'étape (a) s'effectue à une température d'environ 95°C.

13. Procédé suivant la revendication 1, dans lequel la protéase dans l'étape (b) est une endoprotéase.

14. Procédé suivant la revendication 13, dans lequel l'endoprotéase est une protéase acide, neutre ou alcaline.

15. Procédé suivant la revendication 14, dans lequel le pH de la protéine désamidée dans l'étape (b) est ajusté avant hydrolyse.

16. Procédé suivant la revendication 15, dans lequel le pH est ajusté à une valeur d'environ 5,5 à environ 8,5.

17. Procédé suivant la revendication 1, dans lequel l'hydrolyse dans l'étape (b) est effectuée en ajoutant successivement au moins deux protéases.

18. Procédé suivant la revendication 1, dans lequel l'hydrolyse dans l'étape (b) est effectuée en ajoutant simultanément au moins deux protéases.

19. Procédé suivant la revendication 18, dans lequel au moins une des protéases est une exopeptidase.

20. Procédé suivant la revendication 2, dans lequel la désodorisation et la décoloration sont effectuées au moyen de charbon actif.

21. Procédé suivant la revendication 6, dans lequel la protéine de qualité alimentaire est une protéine débarrassée des matières grasses pour supprimer la formation de MCDP en présence d'acide chlorhydrique.

22. Procédé suivant la revendication 1, dans lequel l'acide de qualité alimentaire est choisi dans le groupe consistant en acide chlorhydrique, acide sulfurique et acide phosphorique.
